# EUROPEAN PATENT SPECIFICATION

(11) **EP 3 727 554 B1**
(45) Date of publication and mention of the grant of the patent: **22.01.2025**
(21) Application number: 18830260.8
(22) Date of filing: 20.12.2018
(51) Int. Cl.: A61M 25/10

(54) **MEDICAL BALLOON**
MEDIZINISCHER BALLON
BALLONNET MÉDICAL

(30) Priority: 21.12.2017 IE S20170263
(43) Date of publication of application: 28.10.2020
(73) Proprietor: Aran Biomedical Teoranta, Galway, H91 C2NF (IE)
(72) Inventor: KING, Dean, Galway (IE); HOWE, Michael, Galway (IE); MCCARTHY, David, Galway (IE); DURKIN, Tony, Galway (IE)
(74) Representative: FRKelly
(86) International application number: PCT/EP2018/086232
(87) International publication number: WO 2019/122150

(56) References cited:
- EP-A1- 2 106 820
- WO-A2-2006/034396
- US-A1- 2002 098 307
- US-A1- 2003 204 235
- US-A1- 2005 271 844
- US-A1- 2010 081 992
- US-A1- 2010 318 029

## Description

### FIELD OF THE INVENTION

This invention relates to medical balloon for high pressure angioplasty.

### BACKGROUND OF THE INVENTION

High pressure balloon catheters are used in vascular angioplasty to break open heavily calcified or highly stenotic lesions, including coronary, peripheral, neurovascular and structural heart applications. One example is the use of high pressure balloons for vascular access to arteriovenous shunts used in kidney dialysis, where patients will need repeated access to the shunts. These shunts become highly stenotic over time and require high pressure balloons to open. Other applications include peripheral angioplasty balloons to break open highly calcified vascular lesions, valvuloplasty to break open calcified heart valves in preparation for valve repair/replacement, and high pressure non-compliant balloons used in coronary angioplasty and for coronary stent placement.

A known medical balloon forming process involves blowing a thin film of polymer material (or multiple layers of different polymers) into the shape of a balloon in a thermal forming process. This process has reached the limit because of the design trade-off between low profile and high strength. The state of the art for high pressure balloons involves braiding a textile material over a film blown balloon. However, the balloons are still prone to bursting at high pressure, potentially resulting in the loss of balloon material within the venous system of the patient. PCT Publication No. WO 2006/034396 discloses a surgical balloon that can be woven, knitted or braided and can withstand extreme pressures relative to other surgical balloons. United States Patent Publication No. 2005/271844 discloses a medical device formed from at least one first layer defining the shape of the medical device, the first layer having an inner surface and an outer surface and a web formed with silk fiber provided over at least a portion of the inner surface, the outer surface or both of the first layer. United States Patent Publication No. 2003/204235 discloses an implantable tubular textile prosthesis comprising a biocompatible fabric, the fabric having a textile construction comprising cold drawn PTFE yarns having a substantially uniform denier and high molecular orientation.

### SUMMARY OF THE INVENTION

In accordance with a first embodiment of the present invention there is provided a medical balloon according to claim 1.

The textile tube may be coated with said polymer material on both its inner and outer surfaces.

In a preferred embodiment the textile tube has a longitudinal axis and comprises a plurality of yarn filaments aligned with said longitudinal axis and a plurality of yarn filaments arranged circumferentially around the tube perpendicular to said longitudinal axis.

The textile tube may be formed from multi-filament yarns, mono-filament yarns or a mixture of multi-filament and mono-filament yarns.

The yarns forming the textile tube may range from 5 to 500 denier.

The yarns forming said textile tube may comprise one or more of regenerated silk viscose, nylon, PET, UHMWPE, LCP (liquid crystal polymer), polyurethane, polypropylene, HDPE, pebax, polyester, Rayon, polytetrafluoroethylene (PTFE), polyamide, carbon, glass fibre, cotton, collagen, calcium alginate, bio-absorbable (e.g. Polyglycolic acid (PGA), 100% PGA, Polyglycolic acid-co-poly-L-lactic acid (PGA/PLLA), Poly-L-lactic acid (PLLA), 100% PLLA.

The yarns forming the textile tube range from 0.025mm to 3.0mm in diameter.

At least some of the yarns forming the textile tube are ring spun.

At least some of the yarns forming the textile tube are twisted.

The yarns may comprise a mixture of two or more different materials.

The polymer material coating the textile tube may comprise polyurethane.

According to a further aspect of the present invention there is provided a method according to claim 9.

The method may comprise coating both the inner and outer surfaces of the textile tube with said polymer material.

In a preferred embodiment the method comprises the steps of coating an outer surface of the textile tube with a polymer material, everting the tube and coating the inner surface (now the outer surface) with a polymer material.

In one embodiment the inner and outer surfaces of the textile tube may be coated with said polymer material by a dispersion process.

In an alternative embodiment at least the outer surface of the textile tube is coated with said polymer material by dipping the textile tube in said polymer material in liquid form.

The method may comprise the step of heating the textile tube in order to effect reflow of at least some yarns forming the textile tube.

The textile tube may be located on a mandrel during coating with said polymer material.

The method may comprise the step of heating the mandrel in order to effect reflow of at least some yarns forming the textile tube.

### BRIEF DESCRIPTION OF THE DRAWINGS

A medical balloon and its method of manufacture in accordance with embodiments of the present invention will now be described, by way of example only, with reference to the accompanying drawings, in which :-
Figure 1 illustrates a textile tube forming part of a medical balloon in accordance with an embodiment of the present invention;
Figure 2 illustrates the textile tube from Figure 1 being spray coated with a polymer layer using a dispersion coating machine to seal the outer surface of the textile tube;
Figure 3 illustrates the textile tube of Figure 1 whose outer surface has been completely coated in polymer material;
Figure 4 illustrates the action of completely everting the coated textile balloon in order to expose the uncoated inner surface to the outside. The inner surface, now facing outwards would again be coated as per Figure 2; and
Fig 5 illustrates an alternative method of application of a polymer material to seal the outer surface of the textile tube.

### DETAILED DESCRIPTION OF THE DRAWINGS

The present invention takes an entirely new approach by weaving a seamless textile tube in the shape of a balloon using high strength polymer yarns in a low profile configuration. The textile is subsequently sealed by applying a polymer material using a solution dispersion or dip coating method.

According to an embodiment of the present invention a medical balloon for high pressure vascular angioplasty comprises a seamless textile fabric tube that has been knitted or woven into the shape of a three dimensional cylindrical balloon.

The tube may have fibres/yarns running both axially around the circumference and longitudinally along the length thereof.

The outer and inner surfaces of the textile tube are coated with a polymer material to seal the tube, thus defining an inflatable balloon and allowing the resulting balloon to be inflated.

The resulting medical balloon offers resilient mechanical structure with excellent compliance when inflated to high pressures (i.e. 30 - 50 ATM).

Coating/sealing the textile tube with a polymer material allows the balloon to be inflated as a monolithic structure. By comparison, prior art medical balloons incorporate an already formed / blown balloon where a yarn or textile is then placed over the balloon as a secondary process of offer structure / compliance when inflated.

The textile tube may comprise monofilament yarns, multifilament yarns (which may be heatset in a twisted condition or not) or a combination of both. The textile tube may be formed from ring spun yarns of regenerated silk, nylon/high tension nylon, PET/high tension PET, UHMWPE, LCP (liquid crystal polymer), polyurethane, polypropylene, HDPE, pebax, polyester, Rayon, polytetrafluoroethylene (PTFE), polyamide, carbon, glass fibre, cotton, collagen, calcium alginate, bio-absorbable (e.g. Polyglycolic acid (PGA), 100% PGA, Polyglycolic acid-co-poly-L-lactic acid (PGA/PLLA), Poly-L-lactic acid (PLLA), 100% PLLA.. Yarns can be a mixture of high tension and low melt temperature with the addition of weldable yarns to aid construction. The yarn forming the textile tube may range from 5 to 500 denier. The textile tube may also be formed from a combination of yarns of different materials and/or each yarn may be a combination of fibres of different materials. For example mixing PET with nylon fibres would enable bonding the balloon to other components, as nylon has a relatively low melt temperature compared with PET, which would provide additional strength.

The woven textile can include yarns of Nylon material, such as PA6 or PA12 yarns, to make the balloon compatible with downstream heat bonding processes during catheter assembly to facilitate bonding of the balloon to a catheter shaft.

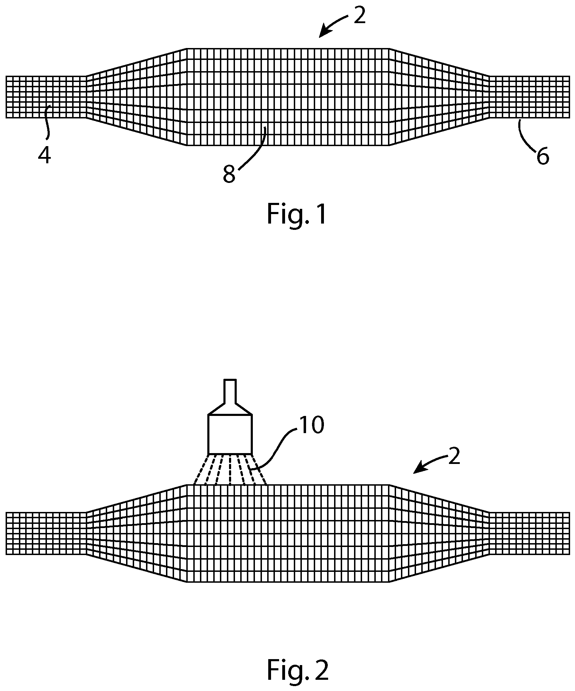

As illustrated in Figures 1 to 4, in a first embodiment the medical balloon in accordance with the present invention may be made by firstly creating the woven textile tube 2 by a suitable weaving process. As shown in Figure 1, the textile tube 2 comprises a longitudinal tubular body having narrowed end regions 4,6 adapted to fit over a catheter shaft and a reinforced central region 8 having a selected diameter to suit the application for which the balloon is intended to be used.

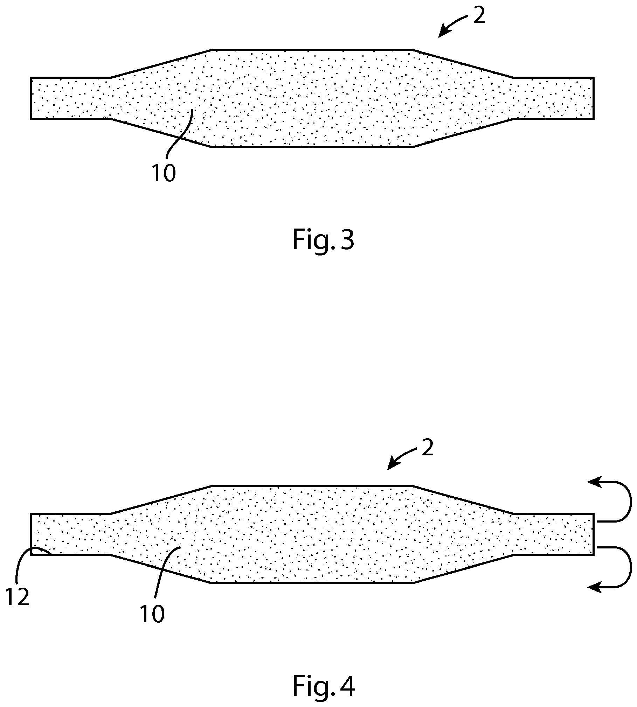

In the embodiment illustrated in Figure 2, the outer surface of the textile tube is coated with a suitable polymer material 10, such as polyurethane, by a dispersion process such as spraying, to seal the tube 2. The tube may be placed over a mandrel during such process. Once the polymer material has dried/cured, the textile tube may be everted (turned inside out), as shown in Figure 4, and the inner surface 12 of the tube, now facing outwards, may be coated with the polymer material by repeating the dispersion process. Once the second coating layer has dried/cured, the resulting balloon may be everted once again to form the finished product.

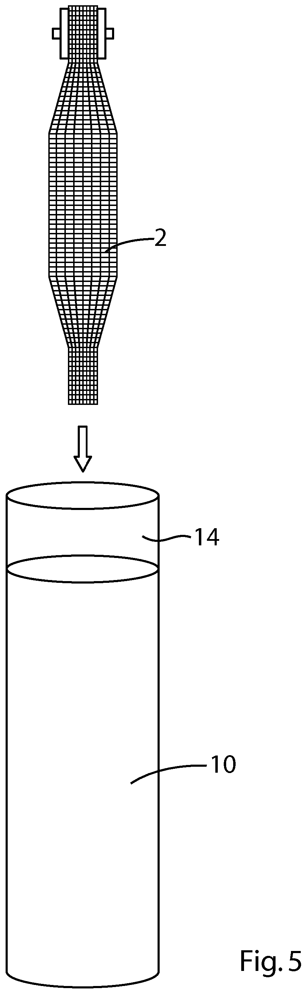

In an alternative embodiment shown in Figure 5 a medical balloon in accordance with the present invention may be made by dip coating the woven textile tube 2 with a polymer material 10. The textile tube 2 may be mounted onto a shaped mandrel and gripped at one end before being dipped into and then withdrawn from a vessel 14 containing the polymer material 10. The textile tube 2 may then be everted as per Figure 4 to dip coat the inner surface of the tube in a further step.

The textile tube 2 may be subjected to a heating step in order to effect reflow of some or all of the yarns forming the textile tube 2, whereby the heat softens the yarns and causes adjacent and/or overlapping yarns to partially melt together in order to increase the integrity and strength of the textile tube 2 and thus ultimately the medical balloon. The heating step may for example be undertaken by heating the mandrel on which the textile tube 2 is mounted for coating with the polymer material 10. Depending on the material and characteristics of the various yarns forming the textile tube 2, the application of heat may result in some of the yarns partially melting and reflowing around other un-melted yarns.

The invention is not limited to the embodiment(s) described herein but can be amended or modified without departing from the scope of the present invention. It is noted that the scope of protection of the current invention is defined by the appended claims.

## Claims

1. A medical balloon, comprising:
a) a plurality of yarn filaments forming a woven textile tube (2) comprising an outer surface and an inner surface, wherein the woven textile tube is characterized as having been heated to cause at least some adjacent and overlapping yarn filaments to partially melt together to increase the integrity and strength of the woven textile tube (2), and
b) wherein yarn filaments forming the woven textile tube range from 5 to 500 denier.

2. The medical balloon of claim 1, further comprising a polymer material (10) coated on at least the outer surface of the woven textile tube (2) in which at least some of the yarn filaments have been partially melted together to seal the tube (2) and thereby define an inflatable balloon.

3. The medical balloon of claim 1, wherein the woven textile tube (2) has a longitudinal axis and comprises a plurality of longitudinal yarn filaments aligned with the longitudinal axis and a plurality of circumferential yarn filaments arranged circumferentially around the tube (2), perpendicular to the longitudinal axis.

4. The medical balloon of any preceding claim, wherein the woven textile tube (2) is formed from multi-filament yarns, mono-filament yarns or a mixture of multi-filament and mono-filament yarns.

5. The medical balloon of any preceding claim, wherein:
a) yarn filaments forming the woven textile tube (2) range from 0.025mm to 3.0mm in diameter; and/or
b) at least some of the yarn filaments forming the woven textile tube (2) are ring spun; and/or
c) at least some of the yarn filaments forming the woven textile tube (2) are twisted.

6. The medical balloon of any preceding claim, wherein yarn filaments forming the textile tube comprise one or more of regenerated silk viscose, nylon, PET, UHMWPE, LCP (liquid crystal polymer), polyurethane, polypropylene, HDPE, pebax, polyester, Rayon, polytetrafluoroethylene (PTFE), polyamide, carbon, glass fibre, cotton, collagen, calcium alginate, bio-absorbable (e.g. Polyglycolic acid (PGA), 100% PGA, Polyglycolic acid-co-poly-L-lactic acid (PGA/PLLA), Poly-L-lactic acid (PLLA), 100% PLLA.

7. The medical balloon of claim 2, wherein the woven textile tube (2) in which at least some of the yarn filaments have been partially melted together is coated with the polymer material (10) on both its inner (12) and outer surfaces.

8. The medical balloon of claim 2, wherein the polymer material (10) coating the woven textile tube (10) in which at least some of the yarn filaments have been partially melted together comprises polyurethane.

9. A method of making a medical balloon, comprising the steps of:
a) weaving a plurality of yarn filaments to form a woven textile tube (2) comprising an outer surface and an inner surface; and
b) heating the woven textile tube (2) to cause at least some adjacent and overlapping yarn filaments to partially melt together,
c) wherein the yarn filaments forming the woven textile tube (2) range from 5 to 500 denier.

10. The method of claim 10, including coating a polymeric material (10) on at least the outer surface of the woven textile tube in which at least some of the yarn filaments have been partially melted together to seal the woven textile tube and thereby define an inflatable balloon.

11. The method of claim 10, including everting the woven textile tube (2) having the polymeric material (10) coated on its outer surface, and then coating the inner surface (12) (now the outer surface) with the polymer material (10).

12. The method of claim 10, including coating at least the outer surface of the woven textile tube (2) in which at least some of the yarn filaments have been partially melted together with the polymer material using a dispersion process.

13. The method of claim 10, including dipping the woven textile tube in which at least some of the yarn filaments have been partially melted together in a liquid polymer material to coat at least the outer surface of the woven textile tube.

14. The method of claim 10, including placing the woven textile tube (2) on a mandrel followed by heating the mandrel to cause adjacent and overlapping yarn filaments to partially melt together and then coating a polymeric material (10) on at least the outer surface of the woven textile tube in which at least some of the yarn filaments have been partially melted together.

15. The method of claim 14, including heating the mandrel to effect reflow of at least some of the yarn filaments around other un-melted yarn filaments forming the woven textile tube (2).

## Patentansprüche

1. Medizinischer Ballon, umfassend:
a) eine Vielzahl von Garnfilamenten, die einen gewebten Textilschlauch (2) bilden, umfassend eine Außenfläche und eine Innenfläche, wobei der gewebte Textilschlauch **dadurch gekennzeichnet ist, dass** er erhitzt wurde, um zu bewirken, dass mindestens einige benachbarte und überlappende Garnfilamente zum Teil miteinander verschmelzen, um die Intaktheit und die Festigkeit des gewebten Textilschlauchs (2) zu erhöhen, und
b) wobei Garnfilamente, die den gewebten Textilschlauch bilden, von 5 bis 500 Denier reichen.

2. Medizinischer Ballon nach Anspruch 1, ferner umfassend ein Polymermaterial (10), das auf mindestens die Außenfläche des gewebten Textilschlauchs (2) beschichtet ist, in dem mindestens einige der Garnfilamente zum Teil miteinander verschmolzen wurden, um den Schlauch (2) zu versiegeln und dadurch einen aufblasbaren Ballon zu definieren.

3. Medizinischer Ballon nach Anspruch 1, wobei der gewebte Textilschlauch (2) eine Längsachse aufweist und eine Vielzahl von Längsgarnfilamenten, die auf die Längsachse ausgerichtet sind, und eine Vielzahl von umlaufenden Garnfilamenten, die umlaufend um den Schlauch (2) senkrecht zur Längsachse angeordnet sind, umfasst.

4. Medizinischer Ballon nach einem der vorhergehenden Ansprüche, wobei der gewebte Textilschlauch (2) aus Multifilamentgarnen, Monofilamentgarnen oder einem Gemisch von Multifilament- und Monofilamentgarnen gebildet wird.

5. Medizinischer Ballon nach einem der vorhergehenden Ansprüche, wobei:
a) Garnfilamente, die den gewebten Textilschlauch (2) bilden, einen Durchmesser von 0,025 mm bis 3,0 mm aufweisen; und/oder
b) mindestens einige der Garnfilamente, die den gewebten Textilschlauch (2) bilden, ringgesponnen sind; und/oder
c) mindestens einige der Garnfilamente, die den gewebten Textilschlauch (2) bilden, verdreht sind.

6. Medizinischer Ballon nach einem der vorhergehenden Ansprüche, wobei Garnfilamente, die den Textilschlauch bilden, eine bzw. eines bzw. einen oder mehrere von regenerierter Seidenviskose, Nylon, PET, UHMWPE, LCP (Flüssigkristallpolymer), Polyurethan, Polypropylen, HDPE, Pebax, Polyester, Rayon, Polytetrafluorethylen (PTFE), Polyamid, Kohlenstoff, Glasfaser, Baumwolle, Kollagen, Calciumalginat, bioloresorbierbarer (z. B. Polyglykolsäure (PGA), 100%iger PGA, Polyglykolsäure-co-Poly-L-Milchsäure (PGA/PLLA), Poly-L-Milchsäure (PLLA), 100%iger PLLA umfassen.

7. Medizinischer Ballon nach Anspruch 2, wobei der gewebte Textilschlauch (2), in dem mindestens einige der Garnfilamente zum Teil miteinander verschmolzen wurden, sowohl auf seiner Innenfläche (12) als auch auf seiner Außenfläche mit dem Polymermaterial (10) beschichtet ist.

8. Medizinischer Ballon nach Anspruch 2, wobei das Polymermaterial (10), das den gewebten Textilschlauch (10) beschichtet, in dem mindestens einige der Garnfilamente zum Teil miteinander verschmolzen wurden, Polyurethan umfasst.

9. Verfahren zur Herstellung eines medizinischen Ballons, umfassend die Schritte:
a) Weben einer Vielzahl von Garnfilamenten, um einen gewebten Textilschlauch (2) zu bilden, der eine Außenfläche und eine Innenfläche umfasst; und
b) Erhitzen des gewebten Textilschlauchs (2), um zu bewirken, dass mindestens einige benachbarte und überlappende Garnfilamente zum Teil miteinander verschmelzen,
c) wobei die Garnfilamente, die den gewebten Textilschlauch (2) bilden, von 5 bis 500 Denier reichen.

10. Verfahren nach Anspruch 10, beinhaltend ein Beschichten eines polymeren Materials (10) auf mindestens die Außenfläche des gewebten Textilschlauchs, in dem mindestens einige der Garnfilamente zum Teil miteinander verschmolzen wurden, um den gewebten Textilschlauch zu versiegeln und dadurch einen aufblasbaren Ballon zu definieren.

11. Verfahren nach Anspruch 10, beinhaltend ein Umstülpen des gewebten Textilschlauchs (2), auf dessen Außenfläche das polymere Material (10) beschichtet ist, und dann ein Beschichten der Innenfläche (12) (jetzt die Außenfläche) mit dem Polymermaterial (10).

12. Verfahren nach Anspruch 10, beinhaltend ein Beschichten mindestens der Außenfläche des gewebten Textilschlauchs (2), in dem mindestens einige der Garnfilamente zum Teil miteinander verschmolzen wurden, zusammen mit dem Polymermaterial v unter Verwendung eines Dispersionsvorgangs.

13. Verfahren nach Anspruch 10, beinhaltend ein Tauchen des gewebten Textilschlauchs, in dem mindestens einige der Garnfilamente zum Teil miteinander verschmolzen wurden, in ein flüssiges Polymermaterial, um die Außenfläche des gewebten Textilschlauchs zu beschichten.

14. Verfahren nach Anspruch 10, beinhaltend ein Anordnen des gewebten Textilschlauchs (2) auf einem Dorn, gefolgt von einem Erhitzen des Dorns, um zu bewirken, dass benachbarte und überlappende Garnfilamente zum Teil miteinander verschmelzen, und dann ein Beschichten eines polymeren Materials (10) auf mindestens die Außenfläche des gewebten Textilschlauchs, in dem mindestens einige der Garnfilamente zum Teil miteinander verschmolzen wurden.

15. Verfahren nach Anspruch 14, beinhaltend ein Erhitzen des Dorns, um einen Rückfluss von mindestens einigen der Garnfilamente um andere, nicht verschmolzene Garnfilamente zu bewirken, die den gewebten Textilschlauch (2) bilden.

## Revendications

1. Ballonnet médical, comprenant :
a) une pluralité de filaments de fil formant un tube textile tissé (2) comprenant une surface extérieure et une surface intérieure, dans lequel le tube textile tissé est caractérisé comme ayant été chauffé pour faire fondre partiellement ensemble au moins certains filaments de fil adjacents et se chevauchant pour augmenter l'intégrité et la résistance du tube textile tissé (2), et
b) dans lequel les filaments de fil formant le tube textile tissé varient de 5 à 500 deniers.

2. Ballonnet médical selon la revendication 1, comprenant également un matériau polymère (10) revêtu sur au moins la surface extérieure du tube textile tissé (2) dans lequel au moins certains des filaments de fil ont été partiellement fondus ensemble pour sceller le tube (2) et ainsi définir ainsi un ballonnet gonflable.

3. Ballonnet médical selon la revendication 1, dans lequel le tube textile tissé (2) présente un axe longitudinal et comprend une pluralité de filaments de fil longitudinaux alignés avec l'axe longitudinal et une pluralité de filaments de fil circonférentiels disposés de manière circonférentielle autour du tube (2), de manière perpendiculaire à l'axe longitudinal.

4. Ballonnet médical selon une quelconque revendication précédente, dans lequel le tube textile tissé (2) est formé de fils à filaments multiples, de fils à filament unique ou d'un mélange de fils à filaments multiples et filament unique.

5. Ballonnet médical selon une quelconque revendication précédente, dans lequel :
a) les filaments de fil formant le tube textile tissé (2) varient de 0,025 mm à 3,0 mm en diamètre ; et/ou
b) au moins certains des filaments de fil formant le tube textile tissé (2) sont filés en anneau ; et/ou
c) au moins certains des filaments de fil formant le tube textile tissé (2) sont torsadés.

6. Ballonnet médical selon une quelconque revendication précédente, dans lequel les filaments formant le tube textile comprennent un ou plusieurs parmi la viscose de soie régénérée, le nylon, le PET, l'UHMWPE, le LCP (polymère à cristaux liquides), le polyuréthane, le polypropylène, le HDPE, le pebax, le polyester, la rayonne, le polytétrafluoroéthylène (PTFE), le polyamide, le carbone, la fibre de verre, le coton, le collagène, l'alginate de calcium, le bio-absorbable (par exemple, acide polyglycolique (PGA), 100 % PGA, acide polyglycolique-co-poly-L-lactique (PGA/PLLA), acide poly-L-lactique (PLLA), 100 % PLLA).

7. Ballonnet médical selon la revendication 2, dans lequel le tube textile tissé (2) dans lequel au moins certains des filaments de fil ont été partiellement fondus ensemble est revêtu du matériau polymère (10) à la fois sur ses surfaces intérieure (12) et extérieure.

8. Ballonnet médical selon la revendication 2, dans lequel le matériau polymère (10) recouvrant le tube textile tissé (10) dans lequel au moins certains des filaments de fil ont été partiellement fondus ensemble comprend du polyuréthane.

9. Procédé de fabrication d'un ballonnet médical, comprenant les étapes de :
a) tissage d'une pluralité de filaments de fil pour former un tube textile tissé (2) comprenant une surface extérieure et une surface intérieure ; et
b) chauffage du tube textile tissé (2) pour faire fondre partiellement ensemble au moins certains filaments de fil adjacents et se chevauchant,
c) dans lequel les filaments de fil formant le tube textile tissé (2) varient de 5 à 500 deniers.

10. Procédé selon la revendication 10, comportant le revêtement d'un matériau polymère (10) sur au moins la surface extérieure du tube textile tissé dans lequel au moins certains des filaments de fil ont été partiellement fondus ensemble pour sceller le tube textile tissé et définir ainsi un ballonnet gonflable.

11. Procédé selon la revendication 10, comportant le retournement du tube textile tissé (2) présentant le matériau polymère (10) revêtu sur sa surface extérieure, puis le revêtement de la surface intérieure (12) (désormais la surface extérieure) avec le matériau polymère (10).

12. Procédé selon la revendication 10, comportant le revêtement d'au moins la surface extérieure du tube textile tissé (2) dans lequel au moins certains des filaments de fil ont été partiellement fondus ensemble avec le matériau polymère à l'aide d'un procédé de dispersion.

13. Procédé selon la revendication 10, comportant l'immersion du tube textile tissé dans lequel au moins certains des filaments de fil ont été partiellement fondus ensemble dans un matériau polymère liquide pour revêtir au moins la surface extérieure du tube textile tissé.

14. Procédé selon la revendication 10, comportant le placement du tube textile tissé (2) sur un mandrin suivi du chauffage du mandrin pour faire fondre partiellement ensemble des filaments de fil adjacents et se chevauchant, puis le revêtement d'un matériau polymère (10) sur au moins la surface extérieure du tube textile tissé dans lequel au moins certains des filaments de fil ont été partiellement fondus ensemble.

15. Procédé selon la revendication 14, comportant le chauffage du mandrin pour refondre au moins certains des filaments de fil autour d'autres filaments de fil non fondus formant le tube textile tissé (2).
